# EUROPEAN PATENT SPECIFICATION

(11) **EP 2 222 864 B1**
(45) Date of publication and mention of the grant of the patent: **18.06.2014**
(21) Application number: 08857536.0
(22) Date of filing: 04.12.2008
(51) Int. Cl.: C12P 7/06

(54) **Process for the manufacture of bioethanol**
Verfahren zur Herstellung von Bioethanol
Procédé de fabrication de bioéthanol

(30) Priority: 04.12.2007 EP 07122320
(43) Date of publication of application: 01.09.2010
(73) Proprietor: Kappe, Jantinus, 7971 CS Havelte (NL)
(72) Inventor: SAUTNER, Karl-Heinz, 74918 Angelbachtal (DE)
(74) Representative: Hatzmann, Martin
(86) International application number: PCT/NL2008/050767
(87) International publication number: WO 2009/072878

(56) References cited:
- EP-A- 1 536 016
- EP-A- 1 626 092
- WO-A-2004/081193
- NL-A- 7 906 735
- US-A- 4 340 446
- US-A- 4 374 705
- US-A1- 2006 003 408
- WINGREN ANDERS ET AL: "Energy considerations for a SSF-based softwood ethanol plant" BIORESOURCE TECHNOLOGY, vol. 99, no. 7, 27 September 2007 (2007-09-27), pages 2121-2131, XP022472726 ISSN: 0960-8524
- LARSSON M ET AL: "Production of ethanol from dilute glucose solutions. A technical-economic evaluation of various refining alternatives" BIOPROCESS ENGINEERING, vol. 15, no. 3, 1996, pages 125-132, XP002479980 ISSN: 0178-515X
- UEDA S ET AL: "PRODUCTION OF ETHANOL FROM RAW CASSAVA STARCH BY A NONCONVENTIONAL FERMENTATION METHOD" BIOTECHNOLOGY AND BIOENGINEERING, WILEY & SONS, HOBOKEN, NJ, US, vol. 23, no. 2, 1 January 1981 (1981-01-01), pages 291-300, XP002380368 ISSN: 0006-3592
- DATABASE WPI Week 200780 Thomson Scientific, London, GB; AN 2007-864287 XP002520351 & JP 2007 124933 A (TSUKISHIMA KIKAI CO LTD) 24 May 2007 (2007-05-24)

## Description

The invention relates to a process for the manufacture of bioethanol.

In view of continuously rising petroleum costs and dependence upon fossil fuel resources, considerable attention has been focused on alternative energy resources. Production of ethanol (CH₃CH₂OH) from biomass is one way to reduce both the consumption of crude oil and environmental pollution. Ethanol can be recovered from renewable resources and represents an important renewable liquid fuel for motor vehicles. The use of gasohol (a mixture of gasoline and ethanol) as an alternative motor fuel has been steadily increasing around the world for a number of reasons. The use of ethanol as fuel can decrease the dependence on fossil fuels such as oil, reduce air pollution, and reduce global climate change carbon dioxide build-up.

Although the burning of bioethanol leads to CO₂ emission, this emission can be at least partly compensated by the assimilation of CO₂ by the crops from which the bioethanol can be obtained. Thus, when burned, ethanol derived from fermentation produces no net increase in carbon dioxide in the atmosphere. Further, unlike gasoline, ethanol is an oxygenated fuel that contains 35 % oxygen, which reduces particulate and NOₓ emissions from combustion. Accordingly, the demand for bioethanol will therefore strongly increase in the coming years.

Conventionally, bioethanol is produced by sugar fermentation or by fermentation of glucose originating from starch. The sugar is mainly recovered from biomass sources, such as fuel or energy crops. These crops are grown specifically for energy use and include corn, maize and wheat crops, waste straw, willow and popular trees, sawdust, reed canary grass, cord grasses, jerusalem artichoke, myscanthus and sorghum plants. There is also ongoing research and development into the use of municipal solid wastes to produce ethanol fuel.

Bioethanol can be advantageously produced from starch. Starch molecules are made up of long chains of glucose moieties. Thus, starchy materials can be fermented after breaking starch molecules into simple glucose molecules. Examples of starchy materials commonly used around the world for bioethanol production include cereal grains, potato, sweet potato, and cassava. Starchy materials require hydrolysis to break down the starch into fermentable sugars. This process is also known as saccharification.

In a conventional process for the production of bioethanol from starch containing feedstock, the feedstock is first ground and then heated in a jet cooker during 5-8 minutes at a temperature of about 120 °C. The pH during this heating step is adjusted to about 6.0. Then the feedstock is stored in a secondary liquefaction step during about 90 minutes at a temperature of approximately 95 °C. Liquefaction is followed by storage in saccharification tanks during 8-10 hours at a temperature of approximately 60 °C. This results in a liquid with a glucose content of about 20 wt.%. Finally, the produced glucose is fermented during about 72 hours at a temperature of about 35 °C. Fermentation is performed at a pH of about 4.5. The fermented product has an ethanol content of about 10 wt.%. The ethanol is subsequently concentrated from this water/ethanol mixture by distillation and fractionation.

This conventional production process is relatively energetically consuming. The relatively high glucose content can have a feedback inhibiting effect on the enzyme activity. Furthermore, due to the relatively low ethanol content of the fermented product, distillation requires a large amount of energy. Furthermore, the distillation of the 10 % ethanol solution requires a large amount of cooling water (about 250 m³ of cooling water per m³ of ethanol).

EP 1 626 092 is directed to a method for producing ethanol by using corn flours, using wet milling prior to saccharification (hydrolysis) and fermentation.

EP 1 536 016 describes a method for the preparation of ethanol from cereals, wherein cereal is grinded after which the ground product is subsequently hydrolyzed and fermented, after which the non-fermentable residue is distilled and further treated.

Wingren Anders et al (Bioresource Technology 99 (2008) 2121-2131) describes a method for producing bioethanol from softwood by steam pretreatment followed by simultaneous saccharification and fermentation.

Larsson et al (Bioprocess Engineering 15 (1996) 125-132) describe several refining steps for the production of ethanol from a dilute glucose solution, including distillation with mechanical vapour recompression.

US 4,374,705 is directed to a process for the distillation of alcohol from a glue-like raw alcohol containing solution using distillation.

US 4,340,446 is directed to a process for the separation and recovery of ethanol from an aqueous fermentate using vapor recompression.

WO 2004/081193 describes a method for producing ethanol from plant material comprising saccharification and fermentation of starch.

Ueda et al (Biotechnology and Bioengineering, Vol. XXIII, Pp 291-299 (1981)) describe the production of ethanol from raw cassava root starch in a one-step process of saccharification and fermentation.

US 2006/0003408 relates to an alpha amylase derived from a strain of Aspergillus kawachi, which has granular starch hydrolyzing activity.

JP 2007 124933 describes a method of pre-processing lignocellulose before carrying out enzymatic hydrolysis.

In view of the increasing demand for bioethanol as biofuel it would be highly advantageous to provide an energetically favourable production process.

Object of the invention is to provide a process for the production of bioethanol, which process is energetically more favourable than the conventional production process described above. Further objects include that the process should be economically favorable and minimize or even eliminate waste streams.

The inventors found that these objects can at least in part be met by providing a bioethanol production process in which the feedstock is ground using a wet milling step before being fermented and in which the fermented product is subjected to mechanical vapour recompression.
Accordingly, in a first aspect the invention is directed to a process for the production of bioethanol comprising:
- hydrolysing starch in a ground feedstock comprising starch, thereby converting at least part of said starch into glucose, thus obtaining a partly hydrolyzed feed; and
- grinding said partly hydrolyzed feed, wherein said grinding comprises wet milling;
- fermenting said glucose to form a product, which product comprises ethanol;
- concentrating said product by mechanical vapour recompression to produce an ethanol product stream.

The inventors found that this process is highly energetically advantageous. In accordance with the invention, the energy consuming jet cooking step is eliminated. In addition, the feed of the distillation has higher alcohol content so that less energy is required for distillation. The mechanical vapour recompression only requires electrical energy to condensate the evaporated liquid.

The concentration of ethanol in the product from the fermentation step is preferably as high as possible, because this minimizes equipment size and compression capacity. In practice good results are obtained with ethanol concentrations of about 15 wt.%, but concentrations of about 9-10 wt.% would still be feasible. The higher concentrations are generally more preferred from an economical point of view.

Furthermore, the invention allows capital savings by eliminating cooking and cooling equipment and enabling smaller distillation equipment. Moreover, the amount of cooling water in the process of the invention is significantly reduced in comparison to the conventional production process by using mechanical vapour recompression. Thus, the invention allows a reduction of the total bioethanol production costs.

The feedstock can be any feedstock comprising starch, such as cereal grains (including wheat, triticale, barley, and rye), potato, sweet potato, maize, sorghum, and cassava (tapioca). Preferably cereal grains are used for the process of the invention, most preferably wheat or maize, because these give a particularly ethanol yield per unit area of farm land.

The feedstock does not have to be dry before being used in the process of the present invention. This is advantageous, because feedstock taken directly from agriculture is often wet, in particular when harvested in a rainy period. According to the present invention, feedstock may thus be directly used when obtained from agriculture without the need of a drying step. This is advantageous, because in this way costs may be saved.

The starch containing feedstock may be ground into small parts in one or more steps during the process of the present invention. It was found that such grinding significantly speeds up hydrolysation and fermentation of the feedstock. Without wishing to be bound by theory, it is believed that by grinding the feedstock first, the starch is better exposed and also the glucose is better accessible to the yeast present in the fermentation so that the yeast can more readily access the sugars and convert them. This minimizes high glucose levels, which is a cause of stress on the yeast. The degree of grinding is preferably such that particles are obtained having a flour like consistency, which corresponds e.g. to an average particle size of 50-250, preferably 60-200 µm. If grains are used as the source of starch, fibrous matter (bran) may be separated off after dry grinding the feedstock.Grinding is preferably conducted in two steps. The ground feedstock may first be crushed. Crushing improves the ability to take-up of water by the feedstock, which allows for a reduced time of the pre-treatment step, if present, as described hereinbelow. After hydrolysis, a wet milling step of the at least partly hydrolyzed ground feedstock is conducted.

Preferably, wet milling is used to further grind the feedstock, preferably after hydrolyzing at least part of the starch in said ground feedstock. During wet milling the feedstock is milled while being wet. It was found that wet milling has several advantages over dry milling. It is more energy efficient; dry milling costs about twice the energy used for wet milling. Furthermore, no dust is produced, which saves costs for dust control. Also, wet milling takes up less space than dry milling, because dry milling requires large storages to dry the feedstock. After the milling step, particles are obtained having a flour like consistency, which corresponds e.g. to an average particle size of 50-250, preferably 60-200 µm.

Wet milling may be conducted at a temperature of 0 - 60 °C. Wet milling is preferably conducted at low temperatures, *viz*. a temperature of 0 - 30 °C, more preferably 5 - 20 °C. It was found that at high temperatures, *viz*. temperatures above 30 °C, clogging of the mill could occur because of fibrous matter (bran) present in the feedstock. In conventional methods, glutes are often removed from the feedstock by a soaking step of at least 3 hours.

Preferably, a rasp is used for wet milling. Particular good results have been obtained by using a rasp such as described in NL-A-7 906 735. Such a rasp is designed and intended for use in dry milling of e.g. potatoes or cassava. However, it has now surprisingly been found that by using this rasp for wet milling, very desirable results were obtained. Alternatively, an Entoleter™ mill may be used. Preferably, a wet milling step is conducted after the hydrolysis step and prior to the fermentation step.

The combination of a crushing step prior to hydrolyzing and a wet milling step after hydrolyzing, but prior to fermenting has been found to be particularly desirable.

When bran is obtained as a separate stream, the bran may be fed to an (anaerobic) digester to produce biogas (*viz.* a CH₄ containing stream). This biogas can be burned and the heat can be used to generate electricity and to heat the entire process. When wheat is used as the source of starch, all energy (heat and electricity) that is required for the bioethanol production process can be obtained from the biogas obtained from the bran, which allows for operating the process entirely self-sufficient.

The ground parts are thereafter mixed with water to form a slurry. The slurry preferably has a concentration of starch of about 10-50 wt.%, more preferably 25-35 wt.%, e.g. about 30 wt.%.

Subsequently, the slurry may be homogenized, e.g. using a Supraton™ homogenizer, optionally after adding already part of the hydrolization enzyme.

After grinding by means of crushing and prior to hydrolysing and fermenting, the feedstock may be pre-treated at a temperature of 45-65 °C, preferably at temperature of 50-60 °C. The feedstock can be kept at this temperature for 0.5-4 hours, preferably 1-3 hours, such as about 2 hours, preferably at a low pH, more preferably at pH=3-4, e.g. at a pH of about 3.5. The main purpose of this high temperature and this low pH is to sterilize the feedstock at least to some extent. This pre-treatment may be conducted in an aqueous environment to allow the feedstock to soak, *viz.* take up water, e.g. in water.

Hydrolysing can advantageously be effected by using an enzyme composition comprising amylases, in particular an α-amylase and a glucoamylase. Particular good results have been obtained with the enzyme composition Stargen™, which is commercially available from Genencor International. This enzyme composition contains *Aspergillus kawachi* α-amylase expressed in Trichoderma *reesei* and a glucoamylase from *Aspergillus niger* that work synergistically to hydrolyse granular starch substrate to glucose. These enzymes can hydrolyse uncooked starch. This allows eliminating the heating step that is used in conventional bioethanol production. Accordingly, the starch can be hydrolysed in one step at lower temperature.

Hydrolysis and yeast fermentation can take place simultaneously in the same reaction vessel. Feedback inhibition of high sugar concentrations on the enzyme activity is avoided, because the glucose concentration is maintained low, in particular 10 wt.% or less, preferably 7 wt.% or less, more preferably 5 wt.% or less. As a consequence a more effective conversion of starch into alcohol and a higher alcohol concentration are achieved. In this step nutrients may be added, in particular nitrogen, e.g. in the form of urea. Because proteins have been removed, adding nitrogen can assist to restore the nitrogen balance. According to the method of the present invention, the enzymes of the hydrolysis step do not have to be removed from the feedstock. Consequently, simultaneous hydrolysis and fermentation may occur in the fermentation step.

Furthermore, other enzymes, in particular proteases and cellulases can be added.

The amount of enzymes applied corresponds to a concentration that is normal for this type of operation.

Fermentation is performed by addition of yeast. A suitable commercially obtainable yeast is Fermentis™ Ethanol Red, which is capable to withstand relatively acid conditions. Because the process of the invention is carried out using a highly concentrated slurry, the fermentor can be compact and consequently the total amount of yeast required can be correspondingly low (number of yeast cells per m3 feed is constant).

Hydrolysis and fermentation is preferably carried out at a temperature of 25-50 °C, more preferably at a temperature of 30-40 °C, even more preferably at a temperature of 32-36 °C. Hydrolysis and fermentation can be carried out for 12-96 hours, preferably 24-72 hours, more preferably 36-60 hours, such as about 48 hours.

In a preferred option, the temperature is allowed to decrease after some time. So for instance, the temperature is first kept at 30-35 °C (e.g. about 32 °C) for 15-25 hours (e.g. 20 hours), after which the temperature is reduced to 25-30 °C (e.g. 28 °C), where it is kept for the remaining time (e.g. up to 76 hours, such as about 28 hours). Cooling can be effected by means of a heat exchanger, which is operated with cold water. Again, without wishing to be bound by theory, it is believed that this stepped temperature program helps to avoid stress for the yeast. Apart from high temperatures, other possible causes of stress for the yeast include too high pH, too high temperature and glucose and/or alcohol concentrations that are too high. One of the important features of the present invention is that all of these factors can be controlled in a much better way as compared with prior art processes.

The pH during hydrolysis and fermentation can be kept in the range of 3.5-5.5, preferably in the range of 4.0-5.0. The pH can be adjusted by the addition of an acid in the fermentation tank. A commonly used acid for adjusting the pH is sulphuric acid. However, also other acids can be used, such as hydrochloric acid, nitric acid. Preferably no organic acids are applied, because many of these, such as lactic acid, may have a negative impact on the yeast efficacy.

The ethanol content of the fermented product is relatively high, preferably at least 15 vol.%, more preferably at least 17 vol.%, even more preferably at least 20 vol.%, up to 22 vol.% or even more.

A high ethanol content of the fermented product is advantageous, because further concentration of the ethanol requires less energy consumption. For example, where the conventional process with separate hydrolysis and fermentation requires distillation of about 12.5 m³ of liquid to obtain 1 m³ of ethanol with a purity of more than 99 %, simultaneous hydrolysis and fermenting only requires distillation of about 6.25 m³ of liquid to obtain 1 m³ of ethanol with a purity of more than 99 %. This corresponds to an energy saving of about 1 300 kWh per m³ of bioethanol during distillation.

After the fermentation step, optionally a step is carried out wherein protein is removed from the fermentation product. This may be also with a mechanical vapour recompression, optionally in combination with a decanter.

Further, significant energy saving is achieved in accordance with the process of the invention by concentrating the fermented product using a mechanical vapour recompression system instead of by conventional distillation. In addition, the heating (e.g. up to 55 °C) can be carried out entirely or almost entirely using heat generated elsewhere in the system, in particular when bran is converted to biogas and/or from the mechanical vapour compression step.

In a mechanical vapour recompression evaporator the energy from the steam it creates is reused, thus eliminating the need for an outside source of heat and cooling. A compressor is used to boost the pressure of steam from the evaporator so that it will condense at a higher temperature. This boost enables the latent heat in the steam to be returned to the evaporator, thus generating more steam. Therefore, the same heat is continuously reused. With power being the only utility, the major operating expense of the mechanical vapour recompression evaporator is the mechanical energy required to operate the compressor.

Hence, in accordance with the present invention the heat that is required in the recompression system to evaporate water and ethanol is obtained by condensing the evaporated liquid by means of compression. A major advantage of the invention is that the amount of cooling water required by the mechanical vapour recompression is much lower than for a conventional distillation. This compensates for the electrical energy that is needed for running the compressor. Where a conventional process with normal distillation requires about 250 m³ of cooling water per m³ of ethanol produced, the process of the invention only requires about 40-50 m³ of cooling water per m³ of ethanol produced. At least part of the heat produced in the recompression system by said mechanical vapour recompression may be used to heat the prefermentation tank, e.g. up to 55-60 °C. This further contributes to saving of energy.

Mechanical vapour recompression can also be used for concentrating the residue. The concentrated product can be sold e.g. as animal feed, optionally after further drying. The concentrated product is very valuable as an animal feed, in particular when wheat is used as a feedstock, because it yields an animal feed that is high in protein content.

The investment costs of installing a mechanical vapour recompression system are comparable to the investment costs of a conventional distillation system. However, the use of a mechanical vapour recompression system allows significant savings in operational costs.

The process according to the invention can be operated in batch, semi-batch and continuous fashion, as is illustrated in the following examples.

### Examples

### Reference Example 1

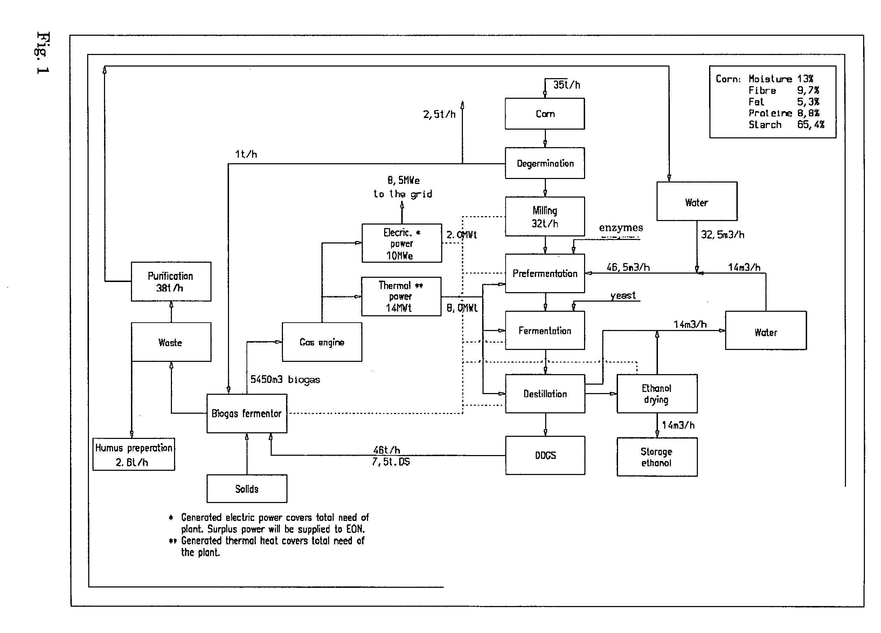

A process as schematically depicted in figure 1 was operated using broken corn.

55.43 kg corn (65% dry matter) was mixed with 46.6 kg water and fed to the prefermenter, as indicated in figure 1. The prefermenter was heated to 40 °C and the pH was adjusted to 3.8 by adding 4M H₂SO₄. Subsequently the temperature was raised to 60 °C, after which 7.2 gram enzyme (Genzyme G998). The mixture was stirred during 2 hours, while the temperature was kept at 60 °C.

A yeast mixture was prepared by dissolving 133.3 g yeast in 400 ml water at a temperature of 30-35 °C. This solution was allowed to stand for an additional 0.5 hours.

Subsequently the yeast mixture was added to the fermentor together with the effluent of the prefermentor. The temperature was lowered to 32 °C and the pH was set at about 3.6 by adding 4M H₂SO₄. Furthermore, 48 g urea, 45 g Stargen™, and 5.4 g GC106 (a protease obtainable from Genencor International) were added.

The entire fermentor contents were allowed to react for 50 hours.

A product stream comprising 16 vol.% ethanol was obtained.

### Reference Example 2

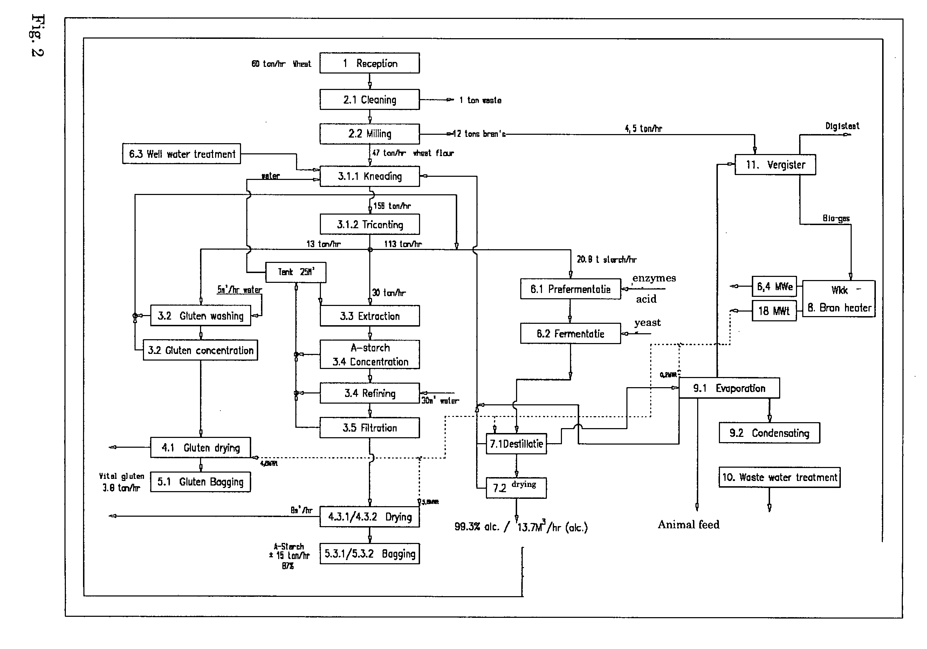

A process as schematically depicted in figure 2 was operated using wheat to illustrate the option wherein bran is used to generate biogas, which is subsequently converted to electricity and heat.

A stream of 60 000 kg/h wheat was cleaned an milled, so that 12 000 kg bran could be separated off. The bran was fed to the biogas fermentor, from which 6.4 MW electricity and 18 MW heat was obtained in a cogeneration plant (combined heat and power, CHP, WKK in figure 2).

This heat and electricity was sufficient to operate the remainder of the process.

The wheat flour was fed to a tricanter and the starch slurry that was obtained therefrom was fed to the prefermentor and fermentor as in the previous example.

The contents of the fermentor had the following composition during its 91 hours of operation.

**Table 1**

| Time / hours | Ethanol concentration / vol.% |
|---|---|
| 0 | 0 |
| 16 | 9.61 |
| 23 | 11.58 |
| 40 | 16.07 |
| 47 | 21.33 |
| 64 | 17.14 |
| 91 | 18.24 |

### Example 3

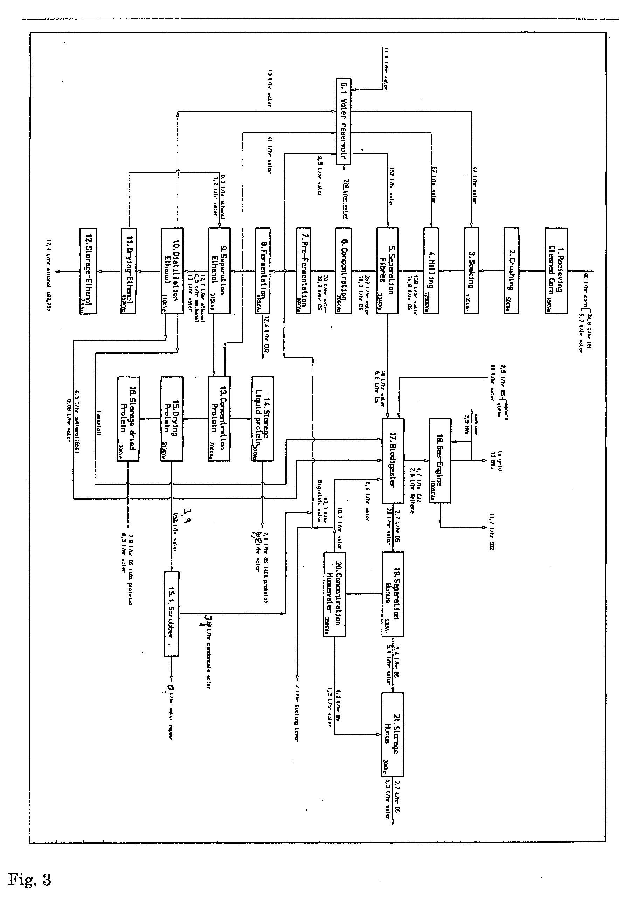

A process as schematically depicted in figure 3 was operated using corn. Hydrolysis is in this example conducted in the soaking step (3). The milling step comprised a wet milling step using a rasp. The process was found to be very energy efficient and resulted in a high yield of ethanol.

## Claims

1. Process for the production of bioethanol comprising:
- hydrolysing starch in a ground feedstock comprising starch, thereby converting at least part of said starch into glucose, thus obtaining a partly hydrolyzed feed and
- grinding said partly hydrolyzed feed, wherein said grinding comprises wet milling; and
- fermenting said glucose to form a product, which product comprises ethanol; and
- concentrating said product by mechanical vapour recompression to produce an ethanol product stream.

2. Process according to claim 1, wherein said hydrolysing is carried out in the presence of an enzyme composition comprising an α-amylase and a glucoamylase.

3. Process according to claim 1 or 2, wherein said fermenting is carried out in the presence of yeast.

4. Process according to any one of the preceding claims, wherein said feedstock is selected from the group consisting of wheat, rye, triticale, barley, potato, sweet potato, maize, sorghum and cassava.

5. Process according to any one of the preceding claims, wherein said wet milling is conducted with a rasp.

6. Process according to any one of the preceding claims, wherein said wet milling is conducted at a temperature of 0 - 30 °C.

7. Process according to any one of the preceding, wherein said wet milling is conducted at a temperature of 5 - 20 °C.

8. Process according to any one of the preceding claims, wherein said grinding further comprises crushing of said starch containing feedstock prior to said hydrolysing.

9. Process according to any one of the preceding claims, wherein said ground feedstock, prior to hydrolysing is subjected to a temperature of 45-65 °C.

10. Process according to claim 9, wherein said ground feedstock is subjected to said temperature for 0.5-4 hours.

11. Process according to claim 9 or 10, wherein said ground feedstock is subjected to said temperature for 1-3 hours.

12. Process according to any one of claims 2-11, wherein said α-amylase is obtained from *Aspergillus kawachi* and/or said glucoamylase is obtained from *Aspergillus niger.*

13. Process according to any one of the preceding claims, wherein the ethanol content of said fermented product is at least 15 wt.%.

14. Process according to any one of the preceding claims, wherein the ethanol content of said fermented product is at least 18 wt.%.

15. Process according to any one of the preceding claims, wherein the ethanol content of said fermented product is at least 20 wt.%.

## Patentansprüche

1. Verfahren zur Herstellung von Bioethanol, umfassend:
- das Hydrolysieren von Stärke zu einem gemahlenen stärkehaltigen Rohstoff, wodurch mindestens ein Teil der Stärke in Glukose umgewandelt wird, um damit einen partiell hydrolysierten Stoff zu erhalten, und
- Mahlen des partiell hydrolysierten Stoffs, wobei das Mahlen die Nassmahlung umfasst; und
- Fermentieren der Glukose, um ein Produkt zu bilden, das Ethanol umfasst; und
- Konzentrieren des Produkts durch mechanische Dampfrekompression, um einen Ethanolproduktstrom zu generieren.

2. Verfahren nach Anspruch 1, wobei das Hydrolysieren in Gegenwart einer Enzymzusammensetzung, die eine α-Amylase und eine Glucoamylase umfasst, ausgeführt wird.

3. Verfahren nach Anspruch 1 oder 2, wobei das Fermentieren in Anwesenheit von Hefe ausgeführt wird.

4. Verfahren nach einem der vorhergehenden Ansprüche, wobei der Rohstoff aus der Gruppe bestehend aus Weizen, Roggen, Triticale, Gerste, Kartoffel, Süßkartoffel, Mais, Sorghum und Cassava, ausgewählt ist.

5. Verfahren nach einem der vorhergehenden Ansprüche, wobei die Nassmahlung mit einer Reibe durchgeführt wird.

6. Verfahren nach einem der vorhergehenden Ansprüche, wobei die Nassmahlung bei einer Temperatur von 0-30 °C durchgeführt wird.

7. Verfahren nach einem der vorhergehenden Ansprüche, wobei die Nassmahlung bei einer Temperatur von 5-20°C durchgeführt wird.

8. Verfahren nach einem der vorhergehenden Ansprüche, wobei das Mahlen ferner das Zerkleinern des stärkehaltigen Rohstoffs vor dem Hydrolysieren umfasst.

9. Verfahren nach einem der vorhergehenden Ansprüche, wobei der gemahlene Rohstoff vor dem Hydrolysieren einer Temperatur von 45-65 °C ausgesetzt wird.

10. Verfahren nach Anspruch 9, wobei der gemahlene Rohstoff der Temperatur für 0,5-4 Stunden ausgesetzt ist.

11. Verfahren nach Anspruch 9 oder 10, wobei der gemahlene Rohstoff der Temperatur für 1-3 Stunden ausgesetzt ist.

12. Verfahren nach einem der Ansprüche 2-11, wobei die α-Amylase von *Aspergillus kawachi* und/oder die Glucoamylase von *Aspergillus niger* erhalten wird.

13. Verfahren nach einem der vorhergehenden Ansprüche, wobei der Ethanolgehalt des fermentierten Produkts mindestens 15 Gew.-% beträgt.

14. Verfahren nach einem der vorhergehenden Ansprüche, wobei der Ethanolgehalt des fermentierten Produkts mindestens 18 Gew.-% beträgt.

15. Verfahren nach einem der vorhergehenden Ansprüche, wobei der Ethanolgehalt des fermentierten Produkts mindestens 20 Gew.-% beträgt.

## Revendications

1. Procédé pour la production de bioéthanol comprenant :
- l'hydrolyse d'amidon dans une matière première alimentaire broyée comprenant de l'amidon, transformant par-là au moins une partie dudit amidon en glucose, obtenant ainsi une alimentation partiellement hydrolysée et
- le broyage de ladite alimentation partiellement hydrolysée, dans lequel ledit broyage comprend un broyage à l'état humide ; et
- la fermentation dudit glucose pour former un produit, lequel produit comprend de l'éthanol ; et
- la concentration dudit produit par recompression de vapeur mécanique pour produire un courant de produit d'éthanol.

2. Procédé selon la revendication 1, dans lequel ladite hydrolyse est réalisée en présence d'une composition d'enzyme comprenant une α-amylase et une glucoamylase.

3. Procédé selon la revendication 1 ou 2, dans lequel ladite fermentation est réalisée en présence de levure.

4. Procédé selon l'une quelconque des revendications précédentes, dans lequel ladite matière première alimentaire est choisie dans le groupe constitué de blé, de seigle, de triticale, d'orge, de pomme de terre, de patate douce, de maïs, de sorgho et de manioc.

5. Procédé selon l'une quelconque des revendications précédentes, dans lequel ledit broyage à l'état humide est réalisé avec une râpe.

6. Procédé selon l'une quelconque des revendications précédentes, dans lequel ledit broyage à l'état humide est réalisé à une température de 0-30°C.

7. Procédé selon l'une quelconque des revendications précédentes, dans lequel ledit broyage à l'état humide est réalisé à une température de 5-20°C.

8. Procédé selon l'une quelconque des revendications précédentes, dans lequel ledit broyage comprend de plus l'écrasement de ladite matière première alimentaire contenant de l'amidon avant ladite hydrolyse.

9. Procédé selon l'une quelconque des revendications précédentes, dans lequel ladite matière première alimentaire broyée avant l'hydrolyse est soumise à une température de 45-65°C.

10. Procédé selon la revendication 9, dans lequel ladite matière première alimentaire broyée est soumise à ladite température pendant 0,5-4 heures.

11. Procédé selon la revendication 9 ou 10, dans lequel ladite matière première alimentaire broyée est soumise à ladite température pendant 1-3 heures.

12. Procédé selon l'une quelconque des revendications 2-11, dans lequel ladite α-amylase est obtenue à partir de *Aspergillus kawachi* et/ou ladite glucoamylase est obtenue à partir *d'Aspergillus niger.*

13. Procédé selon l'une quelconque des revendications précédentes, dans lequel la teneur en éthanol dudit produit fermenté est d'au moins 15 % en masse.

14. Procédé selon l'une quelconque des revendications précédentes, dans lequel la teneur en éthanol dudit produit fermenté est d'au moins 18 % en masse.

15. Procédé selon l'une quelconque des revendications précédentes, dans lequel la teneur en éthanol dudit produit fermenté est d'au moins 20 % en masse.
